# EUROPEAN PATENT SPECIFICATION

(11) **EP 1 244 383 B1**
(45) Date of publication and mention of the grant of the patent: **05.10.2005**
(21) Application number: 00979326.6
(22) Date of filing: 19.12.2000
(51) Int. Cl.: A61B 5/03, A61B 5/00

(54) **DEVICE FOR IN VIVO MEASUREMENT OF PRESSURES AND PRESSURE VARIATIONS IN OR ON BONES**
VORRICHTUNG ZUM IN-VIVO-MESSEN VON DRUCK UND DRUCKSCHWANKUGEN IM ODER AM KNOCHEN
DISPOSITIF POUR LA MESURE IN VIVO DE PRESSIONS ET DE VARIATIONS DE PRESSION DANS OU SUR DES OS

(30) Priority: 07.01.2000 EP 00810012
(43) Date of publication of application: 02.10.2002
(73) Proprietor: Diener AG Precision Machining, 8424 Embrach (CH)
(72) Inventor: AEBLI, Nikolaus, Maorihill, Dunedin (NZ); GRAF, Albert, CH-2572 Mörigen (CH); WUNDERLI, Peter, H., CH-2572 Sutz (CH)
(74) Representative: AMMANN PATENTANWÄLTE AG BERN
(86) International application number: PCT/CH2000/000671
(87) International publication number: WO 2001/049173

(56) References cited:
- DE-A- 3 841 429
- US-A- 3 297 021
- US-A- 4 265 252
- US-A- 4 281 667
- STRECKER W ET AL: "INTRAOPERATIVE MESSUNG VON INTRAMEDULLAREN DRUCKEN MIT EINER MIKRO-SONDE" BIOMEDIZINISCHE TECHNIK,DE,FACHVERLAG SCHIELE UND SCHOEN GMBH. BERLIN, vol. 38, no. 12, 1 December 1993 (1993-12-01), pages 314-316, XP000418357 ISSN: 0013-5585

## Description

The present invention refers to a device for the in vivo measurement of pressures and pressure variations in or on bones or on dental implants according to the preamble of claim 1.

DE-A-38 41 429 discloses a device similar to the preamble of claim 1, however directed solely to measuring the intracranial pressure, where the pressure sensor comprises a membrane whose oscillations act upon an oscillating circuit which measures the variations. The housing of the pressure sensor comprises a circuit board one side of which forms a capacitor with the membrane while a coil is disposed on the other side. The fact to fix all parts of the sensor on both sides of the circuit board attached to a housing renders this sensor inflexible in view of placing the membrane and /or the coil at appropropriate spots. Furthermore, this device involves important operative damages since a hole of 11-12 mm has to be drilled which also causes problems at the removal of the pressure sensor since bone and scar tissue will have covered the pressure sensor in the meantime.

US-A-4 281 667 discloses a method and device for measuring the intracranial differential pressure where a sensor comprising two membranes is implanted in the head and the excursions of the membrane caused by the pressure difference are transmitted by an L-C oscillator to a receiver outside the body wherein the variations are recorded and available at any time. The device is relatively complicated due to the fact that two membranes are necessary, the possible transmission distance is very short, and the location of the membrane, resp. capacitor, is not independent of the location of the coil.

EP-B-579 673 discloses a device for the inspection of implants where a component is fastened to a tooth implant and its natural frequency is detected by an appliance. The component is excited by a signal and examined for mechanical resonance.

Also, various test arrangements are known which allow to detect a loosened prosthesis by means of a vibration analysis where e.g. two connections are established in order to produce vibrations in the bone, on one hand, and to measure the resulting vibrations and to transmit them to an evaluating device, on the other hand.

On the background of this prior art, it is an object of the present invention to provide a device for the measurement of pressures and pressure variations in or on bones or on dental replacements, whose probe has a simple structure and is easy to implant and to remove, on one hand, and which is capable of transmitting signals over such a distance that the latter can be correctly recorded by a reading device outside the human or animal body, on the other hand.
Another object of the invention is to provide such a design of the probe and of the reading device that it is suitable for various tasks inside the human or animal body. These objects are attained by the device as described in claim 1. Further advantages and embodiments are defined in the dependent claims.

The invention is explained in more detail hereinafter with reference to drawings of exemplary embodiments.
- Fig. 1: shows a cross-section according to line I-I in Fig. 2 of a first exemplary embodiment of a probe according to the invention;
- Fig. 2: shows a top view of the probe of Fig. 1;
- Fig. 3: shows a second exemplary embodiment of a probe according to the invention;
- Figs. 4, 4A: schematically show the link between the probe and the receiver of the device;
- Fig. 5: shows the probe of Fig. 1 implanted in a bone;
- Fig. 6: shows an alternative embodiment of the probe of Fig. 5 on an articulation;
- Fig. 7A: schematically shows the probe of Fig. 1 in the medulla of a femur provided with a hip joint prosthesis;
- Fig. 7B: schematically shows an alternative embodiment of the probe of the invention on a hip joint prosthesis implanted in the femur; and
- Fig. 7C: shows an alternative embodiment of Fig. 7B.

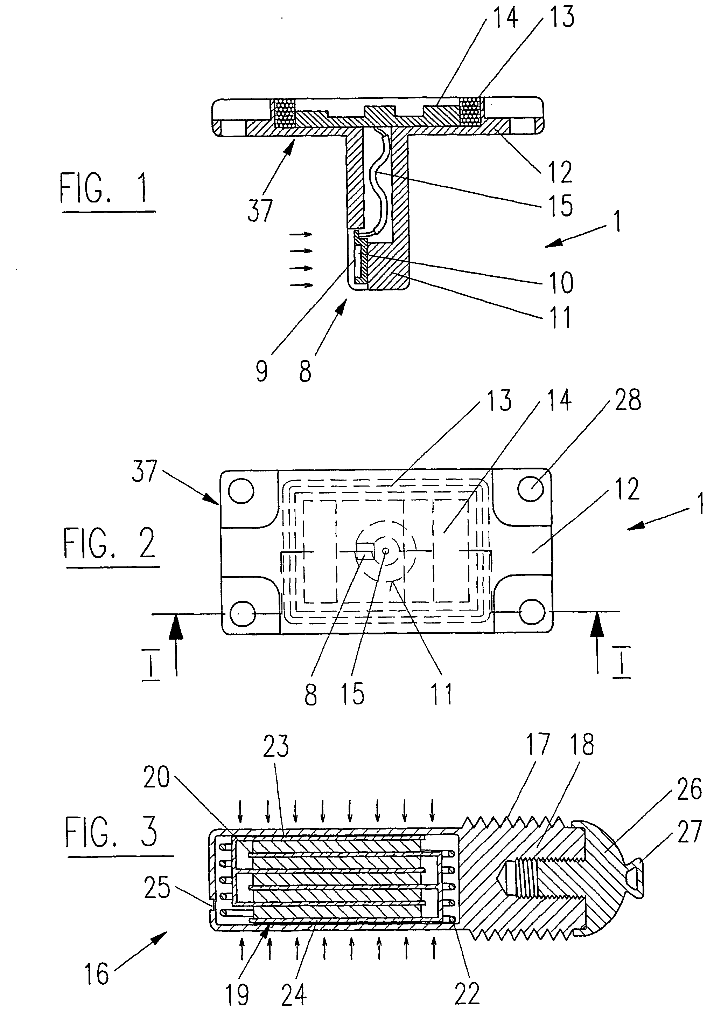

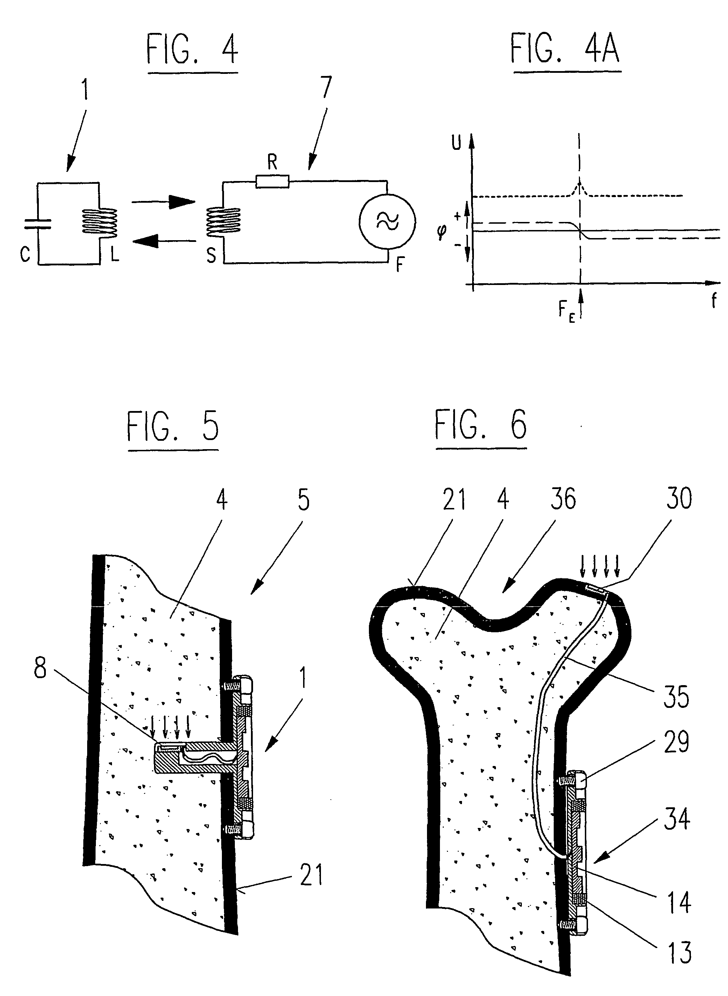

Naturally, a probe intended for implantation should be as small as possible, but at the same time efficient enough to be able to send signals from inside the body to the surface of the body at least. Such a probe is schematically illustrated in Fig. 4, and an exemplary embodiment thereof is shown in Fig. 1. The probe forms an oscillating circuit comprising a capacitor C and a coil L which determine the natural frequency of the oscillating circuit. In this embodiment, coil L is invariable, whereas the membrane of capacitor C is more or less deformed by the pressure outside the probe, thus varying the capacity of the capacitor and therefore also the natural frequency of the oscillating circuit.

The natural frequency is detected and recorded externally, i.e. by means of a receiver outside the body comprising a resonance circuit, thus providing a reliable measuring value of the momentary pressure at the corresponding location.

A first embodiment of the probe is described with reference to Figs. 1 and 2. Probe 1 has a T-shaped cross-section, the stem 11 of housing 37 comprising capacitive pressure sensor 8 and the crosspiece 12 comprising coil 13 and electronic circuit 14, which are connected to each other by an electric conductor 15 extending in the housing as well. The crosspiece further comprises bores 28 for bone screws 29 in order to fasten the probe to the bone.

Pressure sensor 8 shown in Fig. 1 consists of a capacitor comprising a membrane 9, which may be a silicon membrane, for example, and a variation of the distance between the membrane and capacitor surface 10 will result in a variation of the capacity and thus of the natural frequency of the oscillating circuit.

Alternatively, in the example according to Fig. 3, probe 16 may be directly screwed into a bone, more particularly a maxillary, for which purpose it comprises a pin 18 provided with a suitable threaded portion 17 allowing the probe to be screwed into the cortex 21 of the bone. In this embodiment, capacitor 19 is composed of capacitor plates 20 around which a coil 22 is wound. The two outer capacitor plates 23 and 24 are acting as membranes and are fastened to the inside of the housing surfaces, so that a variation of the height of the housing results in a variation of the thickness of the capacitor and thus of its capacity. Advantageously, the housing comprises two thinner portions 25 in order to be more compressible in the direction of its height. If this probe is fastened in the medulla, a cap 26 comprising a fastening loop 27 can be screwed onto threaded pin 18, or a dental replacement resp. a crown can be screwed on in the area of the cap.

In a further embodiment, not shown in the drawings, the membrane, for example made of titanium, is located at the end of the stem, which also can be made of titanium, and pretensioned by the uppermost of several movable parts of a capacitor and acting with reference to the static parts of the capacitor fixed within the stem. The movable parts are guided by a central pin being under the pressure of a spring. A movement of the membrane variates the capacity of the capacitor, which is transmitted like in the first embodiment

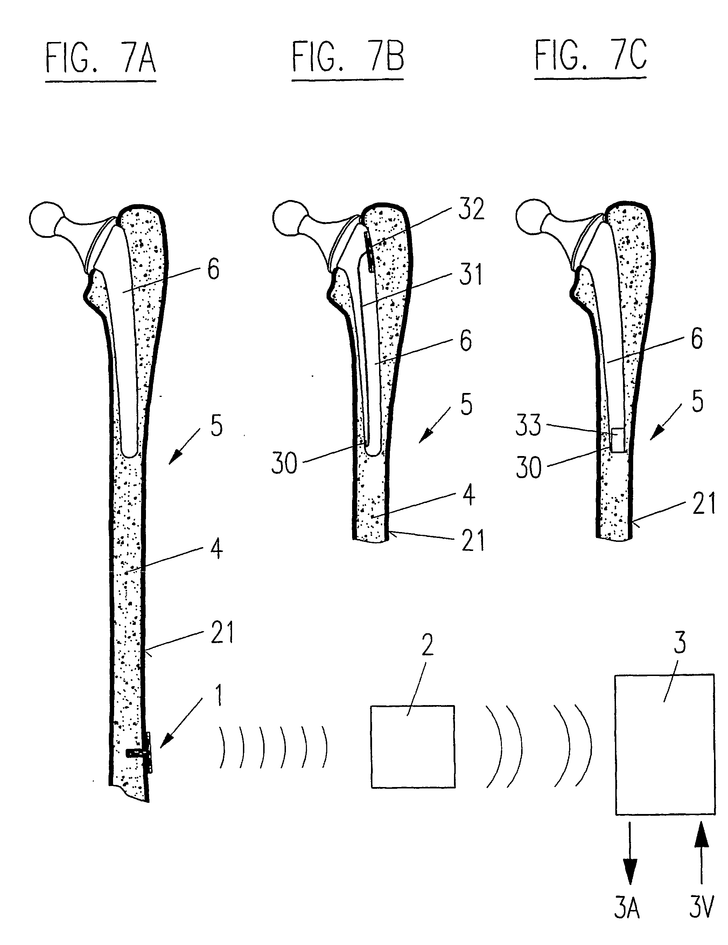

The probes shown in Figs. 1 to 3 and described may be used in this form or in modified forms in a device for the in vivo measurement of pressures and pressure variations. The device of the invention is schematically illustrated in Fig. 7 and is composed of the probe, the so-called interface 2, and of evaluating device 3.

Furthermore, Figs. 4 and 4A schematically illustrate the operation of the measuring device. Fig. 4 only shows the schematic structure of the transmitter/receiver section 7 of the evaluating device, which comprises a frequency generator F, a power supply 3V, an output 3A, see Fig. 7, and a transmitter/receiver coil S which drives the oscillating circuit L-C of the probe and allows to detect the natural frequency Fₑ of the latter in the case of resonance, see Fig. 4A. In the present embodiment, the natural frequency may be in the vicinity of 1450 kHz, and the deviation of the frequency caused by a force of 1 N amounts to approx. 25 kHz. To those skilled in the art, it is apparent that different dimensions of the capacitor and of the coil and different forces will produce different natural frequencies. This measuring arrangement also allows to measure the static pressure, i.e. without a variation of the capacitor dimensions. The above indications shall be regarded as an example.

Such an arrangement further allows an evaluation both of the phase Φ and of the voltage V of the oscillating circuit. In an alternative embodiment, the receiver itself may deliver a D.C. voltage corresponding to the measured pressure, which is subsequently evaluated by means of existing apparatus such as computers, chart recorders, etc.

In view of the increasing tendency towards digital electronic circuits, it is understood that digital signals may be used instead of processing analog signals, thus allowing for an improved measuring accuracy and mainly for an increased insensitivity to interference and longer operating distances.

If the probe and the receiver are used alone, problems may arise due to the fact that the evaluating unit cannot always be positioned close enough to the probe. Therefore, in an advantageous embodiment of the invention according to Fig. 7, a so-called interface 2 is provided which receives the signals of the probe and allows their transmission over a greater distance of e.g. 20 to 100 m. To this end, the interface comprises a power supply e.g. in the form of a long-life battery or of solar cells or the like. The interface may either be operated directly on the body or under the skin in the vicinity of the probe. If the interface is implanted as well, it is obvious that it should be as small as possible and that in this case, according to present knowledge, the power supply should be a long-life battery. The interface is preferably flexible and capable of being externally attached to the skin at the location of the implant by means of a bandage while the measurements are being performed.

Alternatively, the interface may be divided into two component parts which are connected to each other by a cable. In this case, one portion consists of the antenna section, which is e.g. taped to the patient's skin and may be 1-2 mm thick and flexible, while the other portion contains the remaining electronics and the battery. In this manner, a single interface allows to transmit measuring values from a plurality of probes. If it is impossible, for example, to attach an interface to the neck or to another portion of the body because it is too large and uncomfortable, an antenna may be used in these places while the other portion of the interface is affixed to another portion of the patient's body or to the bed.

In the exemplary application according to Fig. 5 or 7A, probe 1 of Fig. 1 is fastened to resp. in a bone. In this case, pressure sensor 8 is positioned inside the medulla 4 of a femur 5 in which the shaft 6 of an artificial hip joint is implanted. Probe 1 is fastened to the bone by means of screws 29, and both coil 13 and electronic circuit 14 are located outside the bone. This solution offers the advantage of allowing a better transmission of the electric values over a greater distance as well as a substantially reduced size of the implantation hole in the bone.

This solution is based upon the realization that the space inside the bone, i.e. the medulla, may be considered as a communicating vessel, such that pressure variations at any point of the medulla will propagate in the latter and compensate each other. Consequently, the same pressure will be measured at any point in the medulla of a bone. Thus, in the present case according to Fig. 7, after the insertion of the prosthesis shaft 6 in the femur 5, the forces acting upon the prosthesis while standing or walking will be transmitted to the medulla and cause an increase of the pressure in the latter. Now, the better the prosthesis is anchored in the bone, the smaller the pressure increase, thereby providing an almost natural, i.e. physiological transformation and transmission of the forces. In other words, the extent of the pressure increase in the medulla is a measure of the quality of the anchorage of a prosthesis, which in turn allows a judgement of the osteo-integration, i.e. of the growth of the bone to the prosthesis or the cement mantle.

The recording of the pressure variations in the medulla allows to monitor the healing progress after the placement of a prosthesis, to determine the maximum allowable load on the prosthesis during convalescence, and to detect the occurrence of complications, especially of a loosening of the prosthesis, at an early stage.

However, the measuring device may also be used for the observation of operations on articulations since pressure variations may be indicative of the course of an operation.

In the embodiment according to Fig. 7B, the probe is divided, i.e. pressure sensor 30 is located at some point of the prosthesis shaft 6 and connected to coil 13 and electronic circuit 14, which are contained in a separate housing, by a longer conductor 31.

In the embodiment according to Fig. 7C, the entire probe 33 is located at the end of the prosthesis shaft.

Considering the example of Fig. 7, it is apparent that a probe may also be implanted in the medulla of other bones, for example at the articulations of the knee, of the shoulder, or of the elbow, and of course also in animals. Furthermore, if the probe is suitably dimensioned, the latter could e.g. be implanted in the oral cavity, i.e. in a tooth or in the jaw bone, in order to monitor the accretion of the bone to a dental implant, for example.

In Fig. 6, the probe is divided as in Fig. 7B, i.e. pressure sensor 30 is connected by an electric conductor 35 to an housing 34 containing coil 13 and electronic circuit 14, the pressure sensor being located at the tibia 36 of a knee joint, and housing 34 being fastened to the bone by means of screws 29.

Furthermore, a probe of this kind also allows to monitor other sequences of motions on human or animal skeletons which may produce a pressure or a pressure variation in the probe, more particularly if the probe is fastened in joints, on the bone, in muscles, tendons or various vessels.

Thus, the probe may be used in veterinary medicine, for example in order to perform a diagnosis of lameness in horses, for training surveillance, or for the classification of suitable soils with regard to stress of the articulations and the development of arthrosis.

In the examples, the probe is illustrated as being rectangular resp. in the form of a cube, but other, especially rounded or spherical shapes are possible as well, particularly if the probe is to be integrated in prostheses, implants, or joints.

In a further development of the invention, a plurality of probes may be used in the same body, the natural frequency of each probe being adjusted such that they operate on adjoining frequency bands. In this case, the receiver resp. the evaluating unit must be designed accordingly.

As already mentioned in the introduction, a probe of the invention may also be used for various other measurements. Thus, the recording of the pressure variations vs. time may be indicative of the development of arthroses. Furthermore, with a single probe or a plurality thereof, it is possible to measure specific pressure loads in joints resp. in parts of joints.

Thus, at least three different applications of the probe can be distinguished, namely:
a) intraossar, as previously described;
b) intraarticular, the probe being fastened between the parts of joints to be measured; and
c) extraossar, the probe being fastened on the outside of bones or in tendons, muscles, or various vessels.

The receiver may also be in the form of a miniaturized multichannel recorder capable of recording, in the receiver itself, the force variations of different probes over prolonged periods of time for ulterior evaluation while each probe can be identified from the outside.

## Claims

1. Device for the in vivo measurement of pressures and pressure variations in or on a body, the device comprising an implantable probe (1, 33) and an evaluating unit (3), the probe comprising an oscillating circuit (L-C) including a pressure sensor (8, 30) in the form of a capacitor having a deformable membrane (9) and a coil (13), and the evaluating unit comprising a resonance oscillating circuit capable of detecting the natural frequency (Fₑ) of the oscillating circuit (7), the natural frequency (Fₑ) of the oscillating circuit (L-C) being determined by the momentary pressure acting on the pressure sensor, or variable according to variations of a dimension of the pressure sensor caused by pressure variations, **characterised in that** the device is foreseen for in vivo measurements inside or outside bones, whereby the membrane (9) is spatially separated independent from the coil (13) and connected to the coil (13) by an electric conductor (15, 31, 35) and **in that** the housing (32, 34; 37) containing at least the coil (13) and the electric circuit (14), is provided with bores (28) and screws (29) in order to be fastened to the cortex (21) of a bone (5, 36).

2. Device according to claim 1, **characterised in that** the pressure sensor (8, 30) is so designed that it is capable of being introduced in the medulla (4) of a bone (5) or attached to a bone (36).

3. Device according to claim 1, **characterised in that** the pressure sensor (8, 30) is so designed that it is capable of being fastened to or integrated in a prosthesis (6).

4. Device according to any one of claims 1 to 3, **characterised in that** the pressure sensor (30) comprises a housing and the coil (13) and the electric circuit (14) are disposed in another housing (32, 34) and connected to each other by an electric conductor (31, 5) to form the probe.

5. Device according to any one of claims 1 to 3, **characterised in that** the pressure sensor (8), the coil (13), and an electric circuit (14) are disposed in a T-shaped housing (37), the pressure sensor (8) being disposed in the stem (11) and the coil (13) as well as the electric circuit (14) being disposed in the crosspiece and connected to each other by an electric conductor (15) to form the probe (1, 33).

6. Device for the in vivo measurement of pressures and pressure variations in or on a body, the device comprising an implantable probe (16) and an evaluating unit (7), the probe (16) comprising an oscillating circuit (L-C) including a pressure sensor in the form of a capacitor (19) having a deformable membrane ((23, 24) and a coil (22), and the evaluating unit (3) comprising a resonance oscillating circuit (7) capable of detecting the natural frequency (Fₑ) of the oscillating circuit, the natural frequency (Fₑ) of the oscillating circuit (L-C) being determined by the momentary pressure acting on the pressure sensor (8), or variable according to variations of a dimension of the pressure sensor (8) caused by pressure variations, **characterised in that** the probe (16) comprises a threaded pin (17, 18) which is designed to be screwed into the cortex (21) of a bone (5) and **in that** the capacitor (19) is contained in a housing and has compressible capacitor plates (20), the two outermost capacitor plates (23, 24) being fastened to the inside of the sides of the housing, which are displaceable relative to each other as well.

7. Device according to claim 6, **characterised in that** the housing comprises thinner portions (25) in order to be compressible and to act on the capacitor (19).

8. Device according to any one of claims 1 to 7, **characterised in that** the device includes an interface (2) which is designed to receive the signals of the probe and to transmit them to the evaluating device, the interface comprising a power supply.

9. Device according to claim 8, **characterised in that** the interface (2) comprises a long-life battery and is designed for implantation.

10. Device according to claim 8 or 9, **characterised in that** the interface (2) is composed of at least two portions which are connected to each other by a cable, one of the portions containing the antenna and the other portion containing the remaining electronics and the battery.

## Patentansprüche

1. Vorrichtung zur In-vivo-Messung von Drücken und Druckschwankungen im oder am Körper, wobei die Vorrichtung eine implantierbare Sonde (1, 33) und eine Auswerteeinheit (3) aufweist, die Sonde einen einen Drucksensor (8, 30) in Form eines Kondensators mit einer verformbaren Membran (9) und eine Spule (13) enthaltenden Schwingkreis (L-C) enthält und die Auswerteeinheit einen Resonanzschwingkreis zur Erkennung der Eigenfrequenz (Fₑ) des schwingkreises (7) aufweist, wobei die Eigenfrequenz (Fₑ) des Schwingkreises (L-C) durch den momentanen Druck auf den Drucksensor bestimmt ist oder durch eine durch Druckschwankungen hervorgerufene Änderung einer Dimension des Drucksensors veränderbar ist, **dadurch gekennzeichnet, dass** die vorrichtung für in-vivo-Messungen innerhalb oder ausserhalb von Knochen vorgesehen ist, wobei die Membran (9) räumlich von der Spule (13) getrennt und von derselben unabhängig und über einen elektrischen Leiter (15, 31, 35) mit der Spule (13) verbunden ist, und dass das Gehäuse (32, 34; 37), welches zumindest die Spule (13) und die elektrische Schaltung (14) enthält, Bohrungen (28) und Schrauben (29) zur Befestigung an der Corticalis (21) eines Knochens (5, 36) aufweist.

2. Vorrichtung nach Anspruch 1, **dadurch gekennzeichnet, dass** der Drucksensor (8, 30) derart ausgebildet ist, dass er in der Medulla (4) eines Knochens (5) anbringbar oder an einem Knochen (36) befestigbar ist.

3. Vorrichtung nach Anspruch 1, **dadurch gekennzeichnet, dass** der Drucksensor (8, 30) derart ausgebildet ist, dass er an einer Prothese (6) befestigbar oder darin integrierbar ist.

4. Vorrichtung nach einem der Ansprüche 1 bis 3, **dadurch gekennzeichnet, dass** der Drucksensor (30) ein Gehäuse aufweist und die Spule (13) und die elektrische Schaltung (14) in einem anderen Gehäuse (32, 34) angeordnet und über einen elektrischen Leiter (31, 5) miteinander verbunden sind und die Sonde bilden.

5. Vorrichtung nach einem der Ansprüche 1 bis 3, **dadurch gekennzeichnet, dass** der Drucksensor (8), die Spule (13) und eine elektrische Schaltung (14) in einem T-förmigen Gehäuse (37) untergebracht sind, wobei der Drucksensor (8) im Schenkel (11) und die Spule (13) sowie die elektrische Schaltung (14) im Querbalken angeordnet und über einen elektrischen Leiter (15) miteinander verbunden sind und die Sonde (1, 33) bilden.

6. Vorrichtung zur In-vivo-Messung von Drücken und Druckschwankungen im oder am Körper, wobei die Vorrichtung eine implantierbare Sonde (16) und eine Auswerteeinheit (7) aufweist, die Sonde (16) einen einen Drucksensor in Form eines Kondensators (19) mit einer verformbaren Membran (23, 24) und eine Spule (22) enthaltenden Schwingkreis (L-C) enthält und die Auswerteeinheit (3) einen Resonanzschwingkreis (7) zur Erkennung der Eigenfrequenz (Fₑ) des Schwingkreises aufweist, wobei die Eigenfrequenz (Fₑ) des Schwingkreises (L-C) durch den momentanen Druck auf den Drucksensor (8) bestimmt ist oder durch eine durch Druckschwankungen hervorgerufene Änderung einer Dimension des Drucksensors (8) veränderbar ist, **dadurch gekennzeichnet, dass** die Sonde (16) einen Gewindestift (17, 18) aufweist, der ausgebildet ist, in der Corticalis (21) eines Knochens (5) eingeschraubt zu werden, und dass der Kondensator (19) in einem Gehäuse enthalten ist und zusammendrückbare Kondensatorplatten (20) aufweist, wobei die beiden äusseren Kondensatorplatten (23, 24) im Innern der ebenfalls relativ zueinander beweglichen Seiten des Gehäuses befestigt sind.

7. Vorrichtung nach Anspruch 6, **dadurch gekennzeichnet, dass** das Gehäuse dünnere Stellen (25) aufweist, so dass es zusammendrückbar ist und auf den Kondensator (19) wirkt.

8. Vorrichtung nach einem der Ansprüche 1 bis 7, **dadurch gekennzeichnet, dass** die Vorrichtung ein Interface (2) aufweist, das ausgebildet ist, die Signale der Sonde aufzunehmen und an die Auswerteeinrichtung weiterzugeben, wobei das Interface eine Stromversorgung aufweist.

9. Vorrichtung nach Anspruch 8, **dadurch gekennzeichnet, dass** das Interface (2) eine Langzeitbatterie aufweist und ausgebildet ist, um implantiert zu werden.

10. Vorrichtung nach Anspruch 8 oder 9, **dadurch gekennzeichnet, dass** das Interface (2) aus mindestens zwei Teilen besteht, welche über ein Kabel miteinander verbunden sind, wobei der eine Teil die Antenne und der andere Teil die restliche Elektronik und die Batterie enthält.

## Revendications

1. Dispositif pour la mesure in vivo de pressions et de variations de pression dans ou au corps, le dispositif comprenant une sonde implantable (1, 33) et une unité d'évaluation (3), la sonde comprenant un circuit oscillant (L-C) composé d'un capteur de pression (8, 30) sous forme d'un condensateur ayant une membrane déformable (9) et d'une bobine (13), et l'unité d'évaluation comprenant un circuit oscillant de résonance pour détecter la fréquence propre (Fₑ) du circuit oscillant (7), la fréquence propre (Fₑ) du circuit oscillant (L-C) étant déterminée par la pression momentanée agissant sur le capteur de pression, ou variable selon les variations d'une dimension du capteur de pression dues à des variations de pression, **caractérisé en ce que** le dispositif est prévu pour des mesures in vivo à l'intérieur ou à l'extérieur d'os, la membrane (9) étant spatialement séparée et indépendante de la bobine (13) et connectée à la bobine (13) par un conducteur électrique (15, 31, 35), et **en ce que** le boîtier (32, 34; 37) logeant au moins la bobine (13) et le circuit électrique (14) est pourvu d'alésages (28) et de vis (29) pour sa fixation dans la corticale (21) d'un os (5, 36).

2. Dispositif selon la revendication 1, **caractérisé en ce que** le capteur de pression (8, 30) est adapté à être introduit dans la moelle (4) d'un os (5) ou attaché à un os (36).

3. Dispositif selon la revendication 1, **caractérisé en ce que** le capteur de pression (8, 30) est adapté à être fixé à ou intégré dans une prothèse (6).

4. Dispositif selon l'une quelconque des revendications 1 à 3, **caractérisé en ce que** le capteur de pression (30) comprend un boîtier et que la bobine (13) et le circuit électrique (14) sont logés dans un autre boîtier (32, 34) et reliés l'une à l'autre par un conducteur électrique (31, 5) pour former la sonde.

5. Dispositif selon l'une quelconque des revendications 1 à 3, **caractérisé en ce que** le capteur de pression (8), la bobine (13) et un circuit électrique (14) sont logés dans un boîtier (37) en forme de T, le capteur de pression (8) étant disposé dans le montant (11) et la bobine (13) ainsi que le circuit électrique (14) étant disposés dans la barre et reliés l'une à l'autre par un conducteur électrique (15) pour former la sonde (1, 33).

6. Dispositif pour la mesure in vivo de pressions et de variations de pression dans ou au corps, le dispositif comprenant une sonde implantable (16) et une unité d'évaluation (7), la sonde (16) comprenant un circuit oscillant (L-C) composé d'un capteur de pression sous forme d'un condensateur (19) ayant une membrane déformable (23, 24) et d'une bobine (22), et l'unité d'évaluation (3) comprenant un circuit oscillant de résonance (7) pour détecter la fréquence propre (Fₑ) du circuit oscillant, la fréquence propre (Fₑ) du circuit oscillant (L-C) étant déterminée par la pression momentanée agissant sur le capteur de pression (8), ou variable selon les variations d'une dimension du capteur de pression (8) dues à des variations de pression, **caractérisé en ce que** la sonde (16) comporte un goujon fileté (17, 18) adapté à être vissé dans la corticale (21) d'un os (5), et **en ce que** le condensateur (19) est logé dans un boîtier et comporte des plaques de condensateur (20) compressibles, les deux plaques de condensateur extérieures (23, 24) étant fixées à l'intérieur des côtés du boîtier, lesquelles sont également déplaçables l'une par rapport à l'autre.

7. Dispositif selon la revendication 6, **caractérisé en ce que** le boîtier présente des parties amincies (25) afin d'être compressible et d'agir sur le condensateur (19).

8. Dispositif selon l'une quelconque des revendications 1 à 7, **caractérisé en ce que** le dispositif comporte une interface (2) adaptée à recevoir les signaux de la sonde et à les transmettre au dispositif d'évaluation, l'interface comprenant une alimentation.

9. Dispositif selon la revendication 8, **caractérisé en ce que** l'interface (2) comporte une pile longue durée et est conçue pour être implantée.

10. Dispositif selon la revendication 8 ou 9, **caractérisé en ce que** l'interface (2) est composée d'au moins deux parties qui sont reliées l'une à l'autre par un câble, l'une des parties logeant l'antenne et l'autre partie logeant le reste de l'électronique et la batterie.
